(19) Europäisches Patentamt
European Patent Office
Office européen des brevets

(11) EP 0 796 836 B1

(12) **EUROPÄISCHE PATENTSCHRIFT**

(45) Veröffentlichungstag und Bekanntmachung des Hinweises auf die Patenterteilung:
**16.08.2000 Patentblatt 2000/33**

(51) Int Cl.[7]: **C07C 45/50**

(21) Anmeldenummer: **97104372.4**

(22) Anmeldetag: **14.03.1997**

(54) **Verfahren zur Herstellung von Aldehyden**

Process for the preparation of aldehydes

Procédé pour la préparation d'aldéhydes

(84) Benannte Vertragsstaaten:
**AT BE CH DE DK ES FI FR GB GR IE IT LI LU NL PT SE**

(30) Priorität: **20.03.1996 DE 19610869**

(43) Veröffentlichungstag der Anmeldung:
**24.09.1997 Patentblatt 1997/39**

(73) Patentinhaber: **Celanese Chemicals Europe GmbH**
**60439 Frankfurt am Main (DE)**

(72) Erfinder: **Bahrmann, Helmut, Dr.**
**46499 Hamminkeln (DE)**

(56) Entgegenhaltungen:
**EP-A- 0 491 239**
**EP-A- 0 571 819**
**EP-A- 0 602 442**
**EP-A- 0 602 463**
**US-A- 5 091 350**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Herstellung von Aldehyden durch Hydroformylierung von höheren Olefinen in Gegenwart von wasserlöslichen Rhodium-Komplexkatalysatoren und Lösungsvermittlern.

Es ist bekannt, durch Umsetzung von Olefinen mit Kohlenmonoxid und Wasserstoff Aldehyde und Alkohole herzustellen. Die Hydroformylierung wird durch Hydridometallcarbonyle, vorzugsweise solcher der Metalle der 8. Gruppe des Periodensystems, katalysiert. Neben Kobalt, das als Katalysatormetall in großem Umfang technische Anwendung findet, gewinnt in letzter Zeit Rhodium zunehmende Bedeutung. Im Gegensatz zu Kobalt gestattet Rhodium, die Reaktion bei niedrigem Druck durchzuführen; darüber hinaus werden vorzugsweise geradkettige n-Aldehyde und nur in untergeordnetem Maße iso-Aldehyde gebildet. Schließlich ist auch die Hydrierung der Olefine zu gesättigten Kohlenwasserstoffen bei Anwendung von Rhodium-Katalysatoren deutlich geringer ausgeprägt als bei Anwendung von Kobalt-Katalysatoren.

Bei den in der Technik eingeführten Verfahren wird der Rhodium-Katalysator in Form von modifizierten Hydridorhodiumcarbonylen eingesetzt, die zusätzlich Liganden enthalten. Meist liegen die Liganden im Überschuß vor, so daß das Katalysatorsystem aus Rhodium-Komplexverbindung und freiem Ligand besteht. Besonders bewährt haben sich als Liganden tertiäre Phosphine oder Phosphite. Ihre Anwendung ermöglicht es, den Reaktionsdruck bei der Hydroformylierung auf Werte unter 30 MPa zu senken.

Probleme wirft bei diesem Verfahren jedoch die Abtrennung der Reaktionsprodukte und die Wiedergewinnung der im Reaktionsprodukt homogen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d.h. Olefinen mit bis zu etwa 6 Kohlenstoffatomen im Molekül beschritten werden. Außerdem hat sich gezeigt, daß die thermische Belastung des Destillationsgutes auch zu erheblichen Katalysatorverlusten durch Zersetzung der Rhodium-Komplexverbindungen führt.

Die geschilderten Mängel werden durch Anwendung von Katalysatorsystemen vermieden, die in Wasser löslich sind. Derartige Katalysatorsysteme sind z.B. in der DE-C-26 27 354, EP-A-0 571 819 und EP-B-0 491 240 beschrieben. Die Löslichkeit der Rhodium-Komplexverbindungen wird hierbei durch Verwendung von sulfonierten oder carboxylierten Triarylphosphinen bzw. sulfonierten Diphosphinen als Komplexbestandteil erreicht. Die Abtrennung des Katalysators vom Reaktionsprodukt nach Beendigung der Hydroformylierungsreaktion erfolgt bei dieser Verfahrensvariante einfach durch Trennung von wäßriger und organischer Phase, d.h. ohne Destillation und damit ohne zusätzliche, thermische Verfahrensschritte.

Diese Hydroformylierung mit wasserlöslichen Katalysatorsystemen hat sich ausgezeichnet für die niederen Olefine Ethylen, Propylen sowie die Butene bewährt. Setzt man höhere Olefine wie Hexen, Octen oder Decen ein, geht der Umsatz der Hydroformylierung merklich zurück, so daß die Wirtschaftlichkeit der Reaktion in technischem Maßstab nicht mehr gegeben ist.

Verursacht wird der Rückgang des Umsatzes durch die Abnahme der Löslichkeit höherer Olefine in Wasser, denn die Reaktion zwischen den Reaktanten läuft in der wäßrigen Phase ab.

Aus der EP-B-0 157 316 ist es bekannt, die Hydroformylierung höherer Olefine bei Vorliegen einer wäßrigen und einer mit ihr nicht oder nur wenig mischbaren organischen Phase in Gegenwart von Lösungsvermittlern durchzuführen. Als Katalysatoren werden trisulfonierte Triarylphosphine enthaltende Rhodium-Komplexverbindungen eingesetzt. Bei den Lösungsvermittlern handelt es sich um kationische Phasentransferreagenzien der Formel

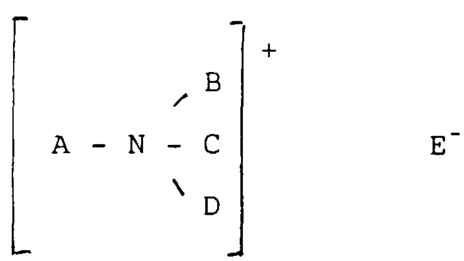

in der A für einen geradkettigen oder verzweigten Alkyl-, ω-Hydroxyalkyl-, Alkoxy- oder einen gegebenenfalls substituierten Arylrest mit jeweils 6 bis 25 Kohlenstoffatomen oder für den Rest $R^7$-CONH-$CH_2$-$CH_2$-$CH_2$-, wobei $R^7$ einen geradkettigen oder verzweigten Alkylrest mit 5 bis 11 Kohlenstoffatomen bedeutet, steht, B, C und D gleich oder verschieden sind und geradkettige oder verzweigte Alkyl- oder ω-Hydroxyalkylreste mit 1 bis 4 Kohlenstoffatomen bedeuten oder C und D zusammen mit N einen heterocyclischen Fünf- oder Sechsring bilden und E für Chlorid, Bromid, Jodid und insbesondere Sulfat, Tetrafluoborat, Acetat, Methosulfat, Benzolsulfonat, Alkylbenzolsulfonat, Toluolsulfonat, Lactat oder Citrat steht, verwendet werden.

Bei der Hydroformylierung von n-Hexen-1 in Gegenwart dieser Lösungsvermittler werden im Vergleich zur Hydroformylierung ohne Lösungsvermittler Umsatzsteigerungen von ca. 20% auf durchschnittlich 40% erreicht. Gleichzeitig verschlechtert sich jedoch in Gegenwart des Lösungsvermittlers das Verhältnis von n-Aldehyd zu den iso-Aldehyden von 98 : 2 (ohne Lösungsvermittler) auf 95 : 5 bis 96 : 4.

Ein Umsatzniveau von ca. 40% ist jedoch für eine technische Anwendung zu niedrig. Durch Optimierung der Reaktionsbedingungen in Form einer Verringerung der zugesetzten Lösungsvermittlermengen kann der Umsatz von 40% auf 70 - 75% angehoben werden, gleichzeitig sinkt aber das n/i-Verhältnis noch weiter auf 91 : 9 ab. Dieser Wert ist vom wirtschaftlichen Aspekt im Hinblick auf die Ausbeute an gewünschtem n-Aldehyd völlig unbefriedigend.

Aus EP-A-0 602 442 ist ebenfalls ein Hydroformylierungsverfahren von höheren Olefinen mit 6 bis 20 Kohlenstoffstomen bekannt, bei dem man mit einer wäßrigen Katalysatorphase arbeitet, in der Rhodiumverbindungen und wasserlösliches Phosphin vorliegen. Abgesättigt werden die anionischen Phosphine durch ein quatäres Ammonium- oder Phosphoniumkation, die die Löslichkeit des organischen Substrates in der wäßrigen katalysatorhaltigen Phase erhöhen. Gemäß der in EP-A-0 602 442 offenbarten Arbeitsweise wird ein Gemisch aus Trinatrium-tri(m-sulfophenyl) phosphin und Tri(trimethyltetradecyl)ammonium-tri(m-sulfophenyl)phosphin eingesetzt, wobei man einen Olefinumsatz von 85% beobachtet. Über Produktivitäts- und Aktivitätswerte wird in EP-A-0 602 442 nichts ausgesagt.

Auch EP-A-0 602 463 offenbart ein Hydroformylierungsverfahren höherer Olefine in Gegenwart einer wäßrigen Katalysatorlösung, die ein wasserlösliches Phosphin sowie als Lösungsvermittler quatäre Phosphoniumverbindungen enthält. Nach der beschriebenen Arbeitsweise führt die Hydroformylierung von Tetradecen-1 in Gegenwart von Trinatrium-tri(m-sulfophenyl)phosphin und Tetradecyl-triethylphosphoniumbromid zu einer Umsatzsteigerung auf einem mäßigen Niveau von 28%, während der Aktivitäts- und Produktivitätswert bei 1,01 mol Aldehyde pro mol Rhodium und Minute bzw. 0,024 g Aldehyd pro $cm^3$ Katalylsatorlösung und Stunde liegt.

Es bestand daher die Aufgabe, ein Verfahren zu entwicklen, das es erlaubt, höhere Olefine in einem aus wäßriger Katalysatorlösung und organischen Ausgangsstoffen und gegebenenfalls Reaktionsprodukten sowie gasförmigen Reaktanten gebildeten Mehrphasensystem mit hohem Umsatz und gleichzeitig hoher Selektivität zum n-Aldehyd zu hydroformylieren.

Die vorstehend beschriebene Aufgabe wird gelöst durch ein Verfahren zur Herstellung von Aldehyden durch Umsetzung von olefinisch ungesättigten Verbindungen mit mindestens 3 Kohlenstoffatomen mit Kohlenmonoxid und Wasserstoff in flüssiger Phase in Gegenwart von Wasser, einem wasserlösliche Rhodium-Komplexverbindungen enthaltenden Katalysatorsystem und Lösungsvermittlern. Es ist dadurch gekennzeichnet, daß die im Katalysatorsystem enthaltenen wasserlöslichen Rhodium-Komplexverbindungen als Liganden Diphosphine der allgemeinen Formel I enthalten,

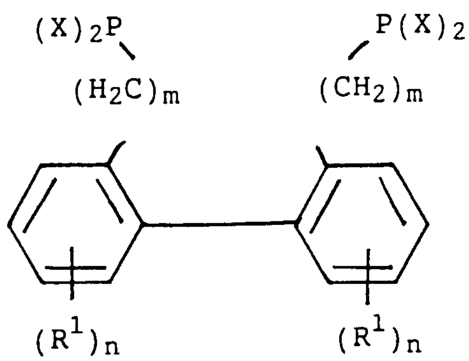

I

die durch eine oder mehrere Sulfonsäuregruppen substituiert sind, wobei X gleich oder verschieden ist und für $C_1$ - $C_9$-Alkyl-, $C_6$ - $C_{10}$-Cycloalkyl-, substituierte oder unsubstituierte $C_6$ - $C_{10}$-Aryl- oder $C_{12}$-Biarylreste steht, $R^1$ gleich oder verschieden ist und Wasserstoff, $C_1$ - $C_{14}$-Alkyl-, $C_1$ - $C_{14}$-Alkoxy-, $C_6$ - $C_{14}$-Cycloalkyl-, $C_6$ - $C_{14}$-Aryl- oder $C_6$ - $C_{14}$-Aroxyreste oder einen anellierten Benzolring bedeutet, m gleich oder verschieden und eine ganze Zahl von 0 bis 5 ist und n ebenfalls gleich oder verschieden ist und für ganze Zahlen von 0 bis 4 steht, und als Lösungsvermittler Verbindungen der allgemeinen Formel II verwendet werden,

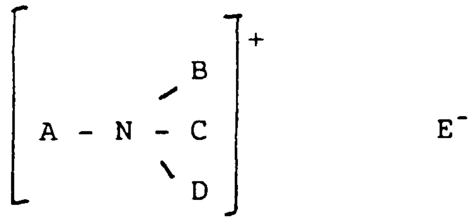

II

wobei A für einen geradkettigen oder verzweigten $C_6$ - $C_{25}$- Alkyl-, $C_6$ - $C_{25}$-ω-Hydroxyalkyl- oder einen gegebenenfalls substituierten $C_6$ - $C_{25}$-Arylrest oder für den Rest $R^7$-CONH-$CH_2$-$CH_2$-$CH_2$, wobei $R^7$ einen geradkettigen oder verzweigten Alkylrest mit 5 bis 11 Kohlenstoffatomen bedeutet, steht, B, C und D gleich oder verschieden sind und geradkettige oder verzweigte Alkyl- oder ω-Hydroxyalkylreste mit 1 bis 4 Kohlenstoffatomen bedeuten oder C und D zusammen mit N einen heterocyclischen Fünf- oder Sechsring bilden und $E^-$ für ein anorganisches oder organisches Anion steht.

Die Hydroformylierung von Olefinen gelingt in Gegenwart von sulfonierten Diphosphinen der allgemeinen Formel I als Bestandteil der Rhodium-Komplexverbindung bei Anwesenheit der genannten Lösungsvermittler der Formel II mit dauerhaft hohem Umsatz und hoher Selektivität zu n-Aldehyden.

In der allgemeinen Formel I steht X vorzugsweise für Phenyl-, Tolyl- oder Naphthylreste, $R^1$ bedeutet vorzugsweise Wasserstoff oder einen Methyl-, Isopropyl-, Isobutyl-, t-Butyl-, Phenyl- oder Naphthylrest oder einen annellierten Benzolring, m ist vorzugsweise 1 und n vorzugsweise 0 oder 1.

$E^-$ steht in Formel II für anorganische Anionen, bevorzugt für ein Halogenidion, für ein Sulfat-, Methosulfat-, Sulfonat- oder Boration. Besonders bevorzugt bedeutet $E^-$ hierbei Chlorid, Bromid, Iodid, Benzosulfonat, $C_7$ - $C_{10}$-Alkylbenzosulfonat, insbesondere Toluolsulfonat oder Tetrafluoroborat.

$E^-$ steht in Formel II ferner für organische Anionen, bevorzugt für ein Carboxylat-, Lactat- oder Citration. Besonders bevorzugt ist hierbei das Acetation.

Besonders bewährt haben sich als Liganden der wasserlöslichen Rhodium-Komplexverbindungen die von der allgemeinen Formel I abgeleiteten sulfonierten 2,2'-Bis(diphenylphosphinomethyl)-1,1'-binaphthyle der Formel III

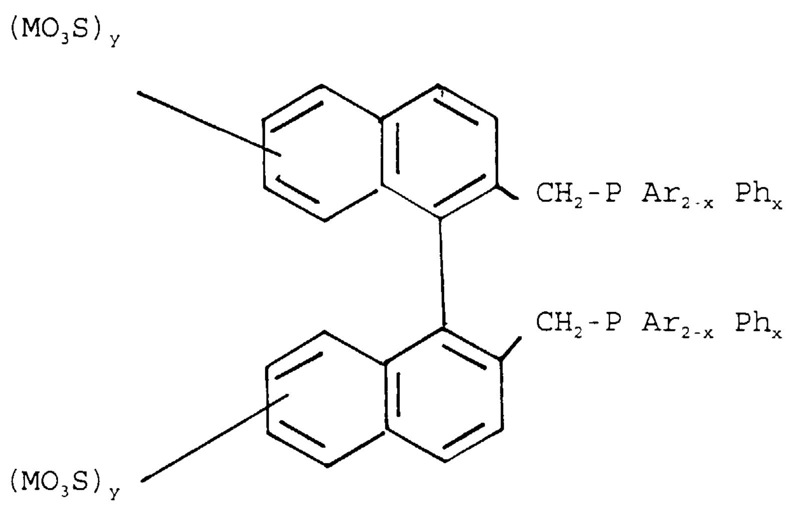

III

in der Ar für m-$C_6H_4SO_3M$, M für Wasserstoff, für Ammonium, ein einwertiges Metall oder das Äquivalent eines mehrwertigen Metalls, bevorzugt für Lithium, Natrium, Kalium oder Barium, und Ph für den Phenylrest steht, y gleich oder verschieden ist und den Wert 1 oder 2, bevorzugt 2 hat und x gleich oder verschieden ist und 0, 1 oder 2, bevorzugt 1 oder 2, bedeutet.

Die Herstellung der sulfonierten Diphosphine der Formeln I und III erfolgt nach den im Stand der Technik üblichen Verfahren, die aus EP-B-0 491 240 sowie EP-A-0 571 819 bekannt sind.

Die Lösungsvermittler der allgemeinen Formel II stellen Stoffe dar, die sowohl mit der wäßrigen, als auch mit der organischen Phase verträglich und insbesondere bei erhöhten Temperaturen in beiden Phasen löslich sind. Derartige Stoffe sind bekannt und werden auch als Phasentransfer-, oberflächenaktives- oder amphiphiles Reagenz oder als Tenside bezeichnet.

Ihre Wirkung besteht vor allem darin, daß sie die physikalischen Eigenschaften der Grenzflächen zwischen den beiden flüssigen Phasen verändern und dadurch den Übergang des organischen Reaktanten in die wäßrige Katalysatorphase erleichtern.

Von besonderer Bedeutung ist in diesem Zusammenhang, daß der Lösungsmittler keinen negativen Einfluß auf die Aktivität des katalytisch wirksamen Metalls hat.

Die eingesetzten Lösungvermittler der allgemeinen Formel II gehören zur Klasse der kationischen Phasentransferreagenzien, wobei A für einen geradkettigen oder verzweigten $C_6$ - $C_{25}$-, bevorzugt $C_8$ - $C_{20}$-, insbesondere $C_{10}$ - $C_{16}$-Alkyl-, $C_6$ - $C_{25}$-, bevorzugt $C_8$ - $C_{20}$-, insbesondere $C_{10}$ - $C_{18}$-ω-Hydroxyalkyl-,$C_6$ - $C_{25}$-, bevorzugt $C_8$ - $C_{20}$-, insbesondere $C_{10}$ - $C_{16}$-Alkoxy- oder einen gegebenenfalls substituierten $C_6$ - $C_{25}$-, bevorzugt $C_6$ - $C_{18}$-, insbesondere $C_6$ - $C_{12}$-Arylrest oder für den Rest $R^7$-CONH-$CH_2$-$CH_2$-$CH_2$, wobei $R^7$ einen geradkettigen oder verzweigten Alkylrest mit 5 bis 11, bevorzugt 4 bis 10, besonders bevorzugt 3 bis 9 Kohlenstoffatomen bedeutet, steht, B, C und D gleich oder verschieden sind und geradkettige oder verzweigte Alkyl- oder ω-Hydroxyalkylreste mit 1 bis 4, bevorzugt 2 bis 3 Kohlenstoffatomen bedeuten oder C und D zusammen mit N einen heterocyclischen Sechsring bilden.

Beispiele für geeignete Kationen $[NABCD]^+$ sind Stearyltrimethylammonium, Phenyltrimethylammonium, Trimethyl-1-phenyl-ammonium, Benzyltrimethylammonium, Cetyltrimethylammonium, Myristyltrimethylammonium, Dodecylpyridinium, Stearylamidomethylpyridinium, Lauryltrimethylammonium, Benzyltriethylammonium, N-(3-trimethylammoniumpropyl)n-heptansäureamidmethosulfat, Dodecyl-tris-β-hydroxyethylammonium oder N-(β-trimethylammoniumpropyl)n-nonansäureamidmethosulfat.

Als Anionen $E^-$ in der allgemeinen Formel II können Chlorid, Bromid, Iodid, Sulfat, Tetrafluoroborat, Acetat, Methosulfat, Benzosulfonat, Alkylbenzolsulfonat, Toluolsulfonat, Lactat oder Citrat verwendet werden. Bevorzugt sind wegen ihres geringen korrosiven Verhaltens die Methosulfate, Sulfonate und Lactate.

Die Konzentration der Lösungsvermittler in der wäßrigen Katalysatorlösung beträgt 0,05 bis 5 Gew.-%, bevorzugt 0,07 - 2 Gew.-% und besonders bevorzugt 0,1 - 0,5 Gew.-%, bezogen auf die Katalysatorlösung.

Der Katalysator kann dem Reaktionssystem präformiert zugesetzt werden. Mit gleich gutem Erfolg läßt er sich aber auch aus den Komponenten Rhodium oder Rhodiumverbindung und der wäßrigen Lösung des Diphosphins der Formel I unter Reaktionsbedingungen im Reaktionsgemisch, also in Gegenwart des Olefins, herstellen. Außer metallischem Rhodium in feinverteilter Form können als Rhodiumquelle wasserlösliche Rhodiumsalze wie Rhodiumchlorid, Rhodiumsulfat, Rhodiumacetat oder in organischen Medien lösliche Verbindungen wie Rhodium-2-ethylhexanoat oder unlösliche Verbindungen wie Rhodiumoxide eingesetzt werden.

Die Rhodiumkonzentration in der wäßrigen Katalysatorlösung beträgt 10 bis 2000 Gew.-ppm, bevorzugt 20 - 300 Gew.-ppm und besonders bevorzugt 40 - 100 Gew.-ppm, bezogen auf die Katalysatorlösung. Das Diphosphin wird in einer solchen Menge eingesetzt, daß auf 1 mol Rhodium 1 bis 50 mol, vorzugsweise 5 bis 15 mol, des Diphosphins kommen.

Der pH-Wert der wäßrigen Katalysatorlösung soll nicht unter 2 liegen. Allgemein stellt man einen pH-Wert von 2 bis 13, vorzugsweise 4 bis 10, ein.

Die Umsetzung des Olefins mit Wasserstoff und Kohlenmonoxid erfolgt bei Temperaturen von 20 bis 150°C, bevorzugt 50 bis 120°C und Drücken von 0,1 bis 20 MPa, bevorzugt 1 bis 10 MPa.

Die Zusammensetzung des Synthesegases, d.h. das Verhältnis von Kohlenmonoxid zu Wasserstoff, kann in weiten Grenzen variiert werden. Im allgemeinen setzt man ein Synthesegas ein, in dem das Volumenverhältnis von Kohlenmonoxid zu Wasserstoff 1 : 1 beträgt oder von diesem Wert nur wenig abweicht.

Die Umsetzung kann sowohl kontinuierlich als auch diskontinuierlich durchgeführt werden.

Das erfindungsgemäße Verfahren wird mit Erfolg bei der Hydroformylierung von olefinisch ungesättigten Verbindungen mit mindestens 3 Kohlenstoffatomen angewendet.

Geeignet sind besonders olefinisch ungesättigte Verbindungen mit 3 bis 20 Kohlenstoffatomen, die eine oder mehrere,innen- und/oder endständige Doppelbindungen besitzen können. Geeignet sind substituierte oder unsubstituierte Alkene mit 3 bis 20 Kohlenstoffatomen, substituierte oder unsubstituierte Diene mit 4 bis 10 Kohlenstoffatomen, substituierte oder unsubstituierte Cycloalkene oder Dicycloalkene mit 5 bis 12 Kohlenstoffatomen im Ringsystem, Ester einer ungesättigten Carbonsäure mit 3 bis 20 Kohlenstoffatomen und eines aliphatischen Alkohols mit 1 bis 18 Kohlenstoffatomen, Ester einer gesättigten Carbonsäure mit 2 bis 20 Kohlenstoffatomen und eines ungesättigten Alkohols mit 2 bis 18 Kohlenstoffatomen, ungesättigte Alkohle oder Ether mit jeweils 3 bis 20 Kohlenstoffatomen oder araliphatische Olefine mit 8 bis 20 Kohlenstoffatomen.

Bei den substituierten oder unsubstituierten Alkenen mit 3 bis 20 Kohlenstoffatomen kann es sich um geradkettige oder verzweigte Alkene mit endständiger oder innenständiger Lage der Doppelbindung handeln. Bevorzugt sind geradkettige Olefine mit 6 bis 18 Kohlenstoffatomen wie n-Hexen-1, n-Hepten-1, n-Octen-1, n-Nonen-1, n-Decen-1, n-Undecen-1, n-Dodecen-1, n-Octadecen-1 und acyclische Terpene. Geeignet sind auch verzweigte Alkene wie Diisobutylen(2,4,4-Trimethylpenten-1), Tripropylen, Tetrapropylen und Dimersol.

Bevorzugte Beispiele für unsubstituierte Diene mit 4 bis 10 Kohlenstoffatomen sind 1,3-Butadien, 1,5-Hexadien und 1,9-Decadien.

Beispiele für substituierte und unsubstituierte Cycloalkene oder Dicycloalkene mit 5 bis 12 Kohlenstoffatomen im Ringsystem sind Cyclohexen, Cyclooсten, Cyclooctadien, Dicyclopentadien und cyclische Terpene wie Limonen, Pinen, Camphoren und Bisabolen.

Beispielhaft für araliphatische Olefine mit 8 bis 20 Kohlenstoffatomen steht Styrol.

Als Beispiele für Ester einer ungesättigten Carbonsäure mit 3 bis 20 Kohlenstoffatomen und eines aliphatischen Alkohols mit 1 bis 18 Kohlenstoffatomen seien die Acrylsäureester und die Methacrylsäureester mit 1 - 18 Kohlenstoffatomen in der Alkohol-Komponente genannt.

Zu den Estern einer gesättigten Carbonsäure mit 2 - 20 Kohlenstoffatomen und eines ungesättigten Alkohols mit 2 - 18 Kohlenstoffatomen gehören die Vinyl- und Allylester mit 2 - 20 Kohlenstoffatomen in der Carbonsäurekomponente.

Zu den ungesättigten Alkoholen und Ethern zählen beispielsweise die Allylalkohole und Vinylether.

In den folgenden Beispielen werden zur Charakterisierung der Leistungsfähigkeit der Katalysatorsysteme neben dem Verhältnis von n-Aldehyd zu i-Aldehyd die Begriffe der "Aktivität", definiert als

$$\frac{\text{mol Aldehyde}}{\text{g-Atom Rh . min}}$$

und der "Produktivität", definiert als

$$\frac{\text{g-Aldehyde}}{\text{cm}^3\ \text{Katalysatorlösung . h}}$$

verwendet. Die Alkohol- und Kohlenwasserstoffbildung ist minimal.

Beispiele 1 - 3 (Vergleich; Komplexligand: Trinatrium-tri(m-sulfophenyl)-phosphin (TPPTS); Zusatz von Lösungsvermittler)

a) Katalysatorpräformierung

In einem 1 l Autoklaven mit Tauchstutzen werden 450 g (419 ml) einer wäßrigen Lösung von TPPTS mit einem Gehalt von 16,4 Gew.-% Salz sowie 300 ppm Rh als Rh-Acetat vorgelegt. Außerdem werden 75,3 g Tetradecyltrimethylammonium-TPPTS-Lösung (5,1 %ig) entsprechend 3,86 g 100 %iges Salz, entsprechend 0,86 % der gesamten TPPTS-Lösung zugegeben. Synthesegas ($CO/H_2$-Vol.-verhältnis 1 : 1) wird bis zu einem Druck von 2,5 MPa aufgepreßt. Danach wird die Reaktionslösung 3 h unter Rühren bei 125°C mit Synthesegas behandelt, wobei sich der aktive Katalysator bildet. Nach dem Abkühlen auf etwa 30°C wird die Rührung abgestellt und nach einer Absetzzeit von 15 Minuten die überschüssige Lösung über den Tauchstutzen herausgedrückt und analysiert. Die restliche Lösung verbleibt im Autoklaven.

b) Hydroformylierung

In die nach a) hergestellte Lösung werden unter Rühren 105 g n-Hexen-1 gepumpt. Bei konstantem Druck von 2,5 MPa wird das Gemisch auf 125°C erhitzt und 3 h bei dieser Temperatur belassen. Danach kühlt man auf 30°C ab und läßt absitzen. Die überstehende organische Phase wird über den Tauchstutzen herausgedrückt; sie wird gewogen (s. Tabelle 1) und gaschromatographisch untersucht. Der Teilschritt b) wird insgesamt zweimal wiederholt, wobei im wesentlichen die gleichen Ergeb

nisse resultieren. Die in der Tabelle 1 aufgeführten Werte für Aktivität und Produktivität beziehen sich auf die im Autoklaven vorhandenen Mengen wäßriger und organischer Phase. Die Hexen-Einsatzmenge wurde dem Autoklaveninnenstand angepaßt.

Tabelle 1

| Beispiele | 1 | 2 | 3 | ø |
|---|---|---|---|---|
| Einsatz n-Hexen-1 (g) | 104 | 103 | 98 | 100,5 |
| Umsatz (% nach GC) | 75 | 72 | 74 | 73 |

Tabelle 1 (fortgesetzt)

| Beispiele | 1 | 2 | 3 | ø |
|---|---|---|---|---|
| n/i-Verhältnis | 91/9 | 91/9 | 91/9 | 91/9 |
| organische Phase (g) | 123,3 | 122,1 | 118 | 119,4 |
| wäßrige Phase im Reaktor (g) | 417 | 411 | 392 | 402,3 |
| Aktivität (mol $C_7$-al*molRh-1min$^{-1}$) | 3,62 | 3,49 | 3,44 | 3,50 |
| Produktivität (g $C_7$-al*cm$^3$Kat.-lösg.-1h$^{-1}$) | 0,079 | 0,076 | 0,075 | 0,076 |

Beispiele 4 - 6 (Vergleich, Komplexligand: sulfoniertes 2,2'-Bis-(diphenylphosphinomethyl)-1,1'-binaphthyl (BINAS), ohne Zusatz eines Lösungsvermittlers).

a) Katalysatorpräformierung

In einem 0,2 l Autoklaven mit Tauchstutzen werden 109 ml (110 g) einer wäßrigen Lösung von BINAS sowie 50 ppm Rh als Rh-Acetat vorgelegt. Synthesegas ($CO/H_2$-Vol.-verhältnis 1 : 1) wird bis zu einem Druck von 2,5 MPa aufgepreßt. Danach wird die Reaktionslösung 3 h unter Rühren bei 122°C mit Synthesegas behandelt, wobei sich der aktive Katalysator bildet. Nach dem Abkühlen auf etwa 30°C wird die Rührung abgestellt und nach einer Absetzzeit von 15 Minuten die überschüssige Lösung über den Tauchstutzen herausgedrückt und analysiert. Die restliche Lösung verbleibt im Autoklaven.

b) Hydroformylierung

In die nach a) hergestellte Lösung werden unter Rühren 34,3 g n-Hexen-1 gepumpt. Bei konstantem Druck von 2,5 MPa wird das Gemisch auf 122°C erhitzt und 3 h bei dieser Temperatur belassen. Danach kühlt man auf 30°C ab und läßt absitzen. Die überstehende organische Phase wird über den Tauchstutzen herausgedrückt; sie wird gewogen und gaschromatographisch untersucht.

Der Teilschritt b) wird insgesamt zweimal wiederholt, wobei im wesentlichen die gleichen Ergebnisse resultieren. Die in der Tabelle 2 aufgeführten Werte für Aktivität und Produktivität beziehen sich auf die im Autoklaven vorhandenen Mengen wäßriger und organischer Phase. Die n-Hexen-1-Einsatzmenge wurde dem Autoklaveninnenstand angepaßt.

Tabelle 2

| Beispiele | 4 | 5 | 6 | ø |
|---|---|---|---|---|
| Einsatz n-Hexen-1 (g) | 34,3 | 34,3 | 34,3 | 34,3 |
| Umsatz (% nach GC) | 33 | 36 | 39 | 36 |
| n/i-Verhältnis | 97,3/2,7 | 99,1/0,9 | 99.1/0.9 | 98.5/1.5 |
| organische Phase (g) | 32,0 | 38,4 | 36,9 | 35,8 |
| wäßrige Phase im Reaktor (g) | 68,5 | 68,5 | 68,5 | 68,5 |
| Aktivität (mol $C_7$-al*molRh-1min$^{-1}$) | 15,2 | 20,1 | 21,3 | 18,9 |
| Produktivität (g $C_7$-al*cm$^3$Kat.-lösg.-1h$^{-1}$) | 0,049 | 0,065 | 0,069 | 0,061 |

Beispiele 7 - 9 (Komplexligand: BINAS, Zusatz eines Lösungsvermittlers)

a) Katalysatorpräformierung

In einem 0,2 l Autoklaven mit Tauchstutzen werden 112 g einer wäßrigen Lösung von BINAS sowie 50 ppm Rh als Rh-Acetat vorgelegt. Außerdem werden 1 g Tetradecyltrimethylammonium-methocarbonatlösung (27,2 %ig) entsprechend 0,272 g 100 %iges Salz, entsprechend 0,241 % der gesamten BINAS-Lösung, zugegeben. Synthesegas ($CO/H_2$-Vol.-verhältnis 1 : 1) wird bis zu einem Druck von 2,5 MPa aufgepreßt. Danach wird die Reaktionslösung 3 h unter Rühren bei 122°C mit Synthesgas behandelt, wobei sich der aktive Katalyator bildet. Nach dem Abkühlen auf etwa 30°C wird die Rührung abgestellt und nach einer Absetzzeit von 15 Minuten die überschüssige Lösung über den Tauchstutzen herausgedrückt und analysiert. Die restliche Lösung verbleibt im Autoklaven.

b) Hydroformylierung

In die nach a) hergestellte Lösung werden unter Rühren 36,7 g n-Hexen-1 gepumpt. Bei konstantem Druck von 25 bar (2.5 . $10^3$ kPa) wird das Gemisch auf 122°C erhitzt und 3 h bei dieser Temperatur belassen. Danach kühlt man auf 30°C ab und läßt absitzen. Die überstehende organische Phase wird über den Tauchstutzen herausgedrückt; sie wird gewogen und gaschromatographisch untersucht. Der Teilschritt b) wird zweimal wiederholt, wobei im wesentlichen die gleichen Ergebnisse resultieren. Die in der Tabelle 3 aufgeführten Werte für Aktivität und Produktivität beziehen sich auf die im Autoklaven vorhandenen Mengen wäßriger und organischer Phase. Die Hexen-Einsatzmenge wurde dem Autoklaveninnenstand angepaßt.

Tabelle 3

| Beispiele | 7 | 8 | 9 | ø |
|---|---|---|---|---|
| Einsatz n-Hexen-1 (g) | 36.5 | 36.5 | 36.5 | 36.5 |
| Umsatz (% nach GC) | 79 | 76 | 77 | 77.3 |
| n/i-Verhältnis | 99/1 | 99/1 | 99/1 | 99/1 |
| organische Phase (g) | 37 | 46 | 44 | 42.3 |
| wäßrige Phase im Reaktor (g) | 73.4 | 73.4 | 73.0 | 73.3 |
| Aktivität (mol $C_7$-al∗molRh-1min$^{-1}$) | 37.3 | 44.1 | 43.9 | 41.8 |
| Produktivität (g $C_7$-al∗cm$^3$Kat.-lösg.-1h$^{-1}$) | 0.130 | 0.154 | 0.153 | 0.146 |

Die Beispiele 7 - 9 zeigen, daß bei Verwendung von BINAS als Komplexligand durch den Zusatz des Lösungsvermittlers Umsatz, Aktivität und Produktivität deutlich erhöht werden, und gleichzeitig die Selektivität genauso hoch ist wie ohne Lösungsvermittlerzusatz.

Beispiele 10 - 12

Die Durchführung erfolgte analog zu den Beispielen 7 - 9, die Präformierung des Katalysators erfolgte allerdings bei 110°C und die Reaktionsdauer der Hydroformylierung wurde von 3 auf 6 h verdoppelt. Die Ergebnisse der Hydroformylierung sind in Tabelle 4 dargestellt.

Tabelle 4

| Beispiele | 10 | 11 | 12 | ø |
|---|---|---|---|---|
| Einsatz n-Hexen-1 (g) | 35 | 35 | 35 | 35 |
| Umsatz (% nach GC) | 85.7 | 84.5 | 82.3 | 84.2 |
| n/i-Verhältnis | 99/1 | 99/1 | 98/2 | 99/1 |
| organische Phase (g) | 38.2 | 43.0 | 41.1 | 40.8 |
| wäßrige Phase im Reaktor (g) | 69.0 | 69.0 | 68.0 | 68.7 |
| Aktivität (mol $C_7$-al∗molRh-1min$^{-1}$) | 22.81 | 24.71 | 16.17 | 21.23 |
| Produktivität (g $C_7$-al∗cm$^3$Kat.-lösg.-1h$^{-1}$) | 0.076 | 0.082 | 0.054 | 0.071 |

Die Beispiele 10 - 12 zeigen, daß bei Verwendung eines Lösungsvermittlers in Gegenwart von BINAS als Komplexligand durch Variation der Reaktionsbedingungen, insbesondere eine Verlängerung der Hydroformylierungsdauer, der Umsatz bei weiterhin exzellentem n/i-Verhältnis noch deutlich gesteigert werden kann.

**Patentansprüche**

1. Verfahren zur Herstellung von Aldehyden durch Umsetzung von olefinisch ungesättigten Verbindungen mit mindestens 3 Kohlenstoffatomen mit Kohlenmonoxid und Wasserstoff in flüssiger Phase in Gegenwart von Wasser, einem wasserlösliche Rhodium-Komplexverbindungen enthaltenden Katalysatorsystem und Lösungsvermittlern, dadurch gekennzeichnet, daß die im Katalysatorsystem enthaltenen wasserlöslichen Rhodium-Komplexverbindungen als Liganden Diphosphine der allgemeinen Formel I enthalten,

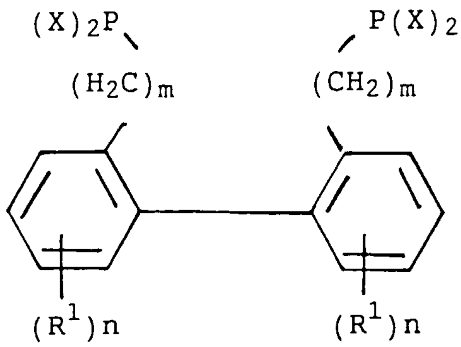

I

die durch eine oder mehrere Sulfonsäuregruppen substituiert sind, wobei X gleich oder verschieden ist und für $C_1$ - $C_9$-Alkyl-, $C_6$ - $C_{10}$-Cycloalkyl-, substituierte oder unsubstituierte $C_6$ - $C_{10}$-Aryl- oder $C_{12}$-Biarylreste steht, $R^1$ gleich oder verschieden ist und Wasserstoff, $C_1$ - $C_{14}$-Alkyl-, $C_1$ - $C_{14}$-Alkoxy-, $C_6$ - $C_{14}$-Cycloalkyl-, $C_6$ - $C_{14}$-Aryl- oder $C_6$ - $C_{14}$-Aroxyreste oder einen anellierten Benzolring bedeutet, m gleich oder verschieden und eine ganze Zahl von 0 bis 5 ist und n ebenfalls gleich oder verschieden ist und für ganze Zahlen von 0 bis 4 steht, und als Lösungsvermittler Verbindungen der allgemeinen Formel II verwendet werden,

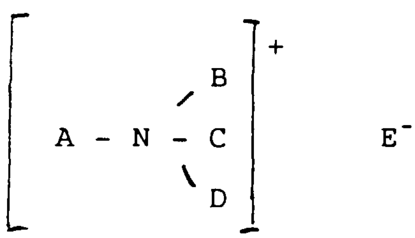

II

wobei A für einen geradkettigen oder verzweigten $C_6$ - $C_{25}$ Alkyl-, $C_6$ - $C_{25}$-ω-Hydroxyalkyl- oder einen gegebenenfalls substituierten $C_6$ - $C_{25}$-Arylrest oder für den Rest $R^7$-CONH-$CH_2$-$CH_2$-$CH_2$, wobei $R^7$ einen geradkettigen oder verzweigten Alkylrest mit 5 bis 11 Kohlenstoffatomen bedeutet, steht, B, C und D gleich oder verschieden sind und geradkettige oder verzweigte Alkyl- oder -Hydroxyalkylreste mit 1 bis 4 Kohlenstoffatomen bedeuten oder C und D zusammen mit N einen heterocyclischen Fünf- oder Sechsring bilden und $E^-$ für ein anorganisches oder organisches Anion steht.

2. Verfahren nach Anspruch 1, dadurch gekennzeichnet, daß in der allgemeinen Formel I X gleich oder verschieden ist und für Phenyl-, Tolyl- oder Naphthylreste steht und $R^1$ Wasserstoff, einen Methyl-, Isopropyl-, Isobutyl-, t-Butyl-, Phenyl- oder Naphthylrest bedeutet, m gleich 1 und n gleich 0 oder 1 ist.

3. Verfahren nach Anspruch 1 oder 2, dadurch gekennzeichnet, daß in der allgemeinen Formel II A für einen geradkettigen oder verzweigten $C_8$ - $C_{20}$-, insbesondere $C_{10}$ - $C_{16}$-Alkyl-, $C_8$ - $C_{20}$-, insbesondere $C_{10}$ - $C_{18}$-ω-Hydroxyalkylkyl- oder einen gegebenenfalls substituierten $C_6$ - $C_{18}$-, insbesondere $C_6$ - $C_{12}$-Arylrest oder für den Rest $R^7$-CONH-$CH_2$-$CH_2$-$CH_2$-, wobei $R^7$ einen geradkettigen oder verzweigten Alkylrest mit 4 bis 10, insbesondere 3 bis 9 Kohlenstoffatomen bedeutet, steht, B, C und D gleich oder verschieden sind und geradkettige oder verzweigte Alkyl- oder ω-Hydroxyalkylreste mit 2 bis 3 Kohlenstoffatomen bedeuten oder C und D zusammen mit N einen heterocyclischen Sechsring bilden.

4. Verfahren nach einem oder mehreren der Ansprüche 1 bis 3, dadurch gekennzeichnet, daß $E^-$ in Formel II für ein Halogenid-, Sulfat-, Methosulfat-, Sulfonat- oder Boration steht.

5. Verfahren nach Anspruch 4, dadurch gekennzeichnet, daß $E^-$ in Formel II für Chlorid, Bromid, Iodid, Benzosulfonat, $C_7$ - $C_{10}$-Alkylbenzosulfonat, insbesondere Toluolsulfonat oder Tetrafluoroborat steht.

6. Verfahren nach einem oder mehreren der Ansprüche 1 bis 3, dadurch gekennzeichnet, daß $E^-$ in Formel II für ein Carboxylat-, bevorzugt Acetation-, für ein Lactat- oder ein Citration steht.

7. Verfahren nach einem oder mehreren der Ansprüche 1 und 3 - 6, dadurch gekennzeichnet, daß als Liganden der wasserlöslichen Rhodium-Komplexverbindungen sulfonierte 2,2'Bis(diphenylphosphino-methyl)-1,1'-binaphthyle der Formel III verwendet werden,

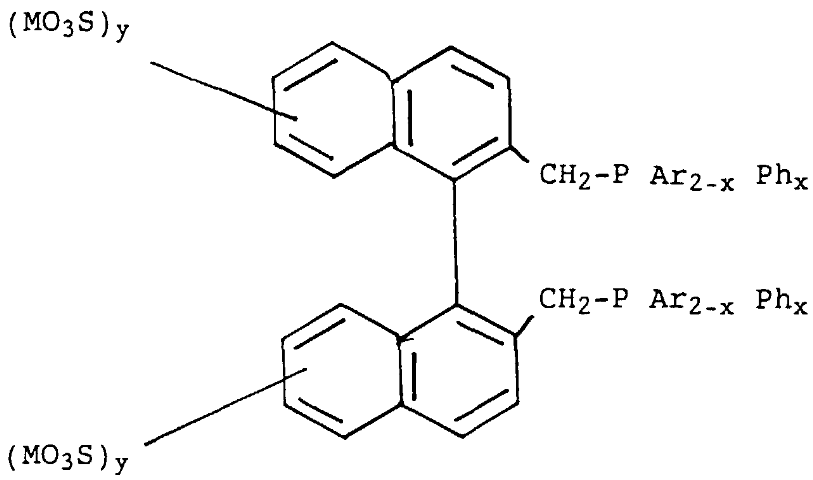

III

in der Ar für m-$C_6H_4SO_3M$, M für Wasserstoff, für Ammonium, ein einwertiges Metall oder das Äquivalent eines mehrwertigen Metalls, bevorzugt für Lithium, Natrium, Kalium oder Barium, und Ph für den Phenylrest steht, y gleich oder verschieden ist und den Wert 1 oder 2, bevorzugt 2 hat und x gleich oder verschieden ist und den Wert 0, 1 oder 2, bevorzugt 1 oder 2, hat.

8. Verfahren nach einem oder mehreren der Ansprüche 1 bis 7, dadurch gekennzeichnet, daß die Konzentration des Lösungsvermittlers in der wäßrigen Katalysatorlösung 0,05 bis 5 Gew.-%, bevorzugt 0,07 - 2 Gew.-% und besonders bevorzugt 0,1 - 0,5 Gew.-%, bezogen auf die Katalysatorlösung, beträgt.

9. Verfahren nach einem der mehreren der Ansprüche 1 bis 8, dadurch gekennzeichnet, daß die Rhodiumkonzentration in der wäßrigen Katalysatorlösung 10 bis 2000 Gew.-ppm, bevorzugt 20 bis 300 Gew.-ppm und besonders bevorzugt 40 bis 100 Gew.-ppm, bezogen auf die Katalysatorlösung, beträgt und pro mol Rhodium 1 - 50 mol, bevorzugt 5 - 15 mol, Diphosphin eingesetzt werden.

10. Verfahren nach einem oder mehreren der Ansprüche 1 bis 9, dadurch gekennzeichnet, daß die Umsetzung bei Temperaturen von 20 bis 150°C, bevorzugt 50 - 120°C, und Drücken von 0,1 bis 20 MPa, bevorzugt 1 - 10 MPa erfolgt.

11. Verfahren nach einem oder mehreren der Ansprüche 1 - 10, dadurch gekennzeichnet, daß als olefinisch ungesättigte Verbindungen substituierte oder unsubstituierte Alkene mit 3 bis 20 Kohlenstoffatomen, substituierte oder unsubstituierte Diene mit 4 bis 10 Kohlenstoffatomen, substituierte oder unsubstituierte Cycloalkene oder Dicycloalkene mit 5 bis 12 Kohlenstoffatomen im Ringsystem, Ester einer ungesättigten Carbonsäure mit 3 bis 20 Kohlenstoffatomen und eines aliphatischen Alkohols mit 1 bis 18 Kohlenstoffatomen, Ester einer gesättigten Carbonsäure mit 2 bis 20 Kohlenstoffatomen und eines ungesättigten Alkohols mit 2 bis 18 Kohlenstoffatomen, ungesättigte Alkohole oder Ether mit jeweils 3 bis 20 Kohlenstoffatomen oder araliphatische Olefine mit 8 bis 20 Kohlenstoffatomen eingesetzt werden.

## Claims

1. A process for preparing aldehydes by reacting olefinically unsaturated compounds having at least 3 carbon atoms with carbon monoxide and hydrogen in the liquid phase in the presence of water, solubilizers and a catalyst system

comprising water-soluble rhodium complexes, wherein the water-soluble rhodium complexes present in the catalyst system contain as ligands diphosphines of the formula I

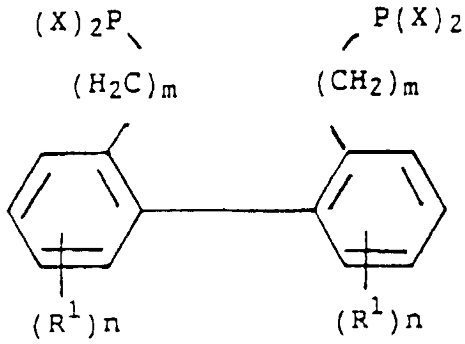

I

which are substituted by one or more sulfonic acid groups, where X are identical or different and are $C_1$-$C_9$-alkyl, $C_6$-$C_{10}$-cycloalkyl, substituted or unsubstituted $C_6$-$C_{10}$-aryl or $C_{12}$-biaryl radicals, $R^1$ are identical or different and are hydrogen, $C_1$-$C_{14}$-alkyl, $C_1$-$C_{14}$-alkoxy, $C_6$-$C_{14}$-cycloalkyl, $C_6$-$C_{14}$-aryl or $C_6$-$C_{14}$-aroxy radicals or a fused-on benzene ring, m are identical or different and are each an integer from 0 to 5 and n are likewise identical or different and are integers from 0 to 4, and the solubilizers used are compounds of the formula II

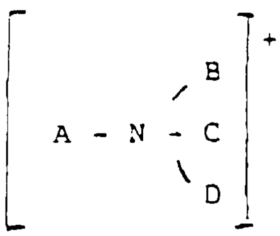

$E^-$ II

where A is a straight-chain or branched $C_6$-$C_{25}$-alkyl, $C_6$-$C_{25}$-ω-hydroxyalkyl or a substituted or unsubstituted $C_6$-$C_{25}$-aryl radical or the radical $R^7$-CONH-$CH_2$-$CH_2$-$CH_2$, where $R^7$ is a straight-chain or branched alkyl radical having from 5 to 11 carbon atoms, B, C and D are identical or different and are straight-chain or branched alkyl or ω-hydroxyalkyl radicals having from 1 to 4 carbon atoms or C and D together with N form a heterocyclic five- or six-membered ring and $E^-$ is an inorganic or organic anion.

**2.** The process as claimed in claim 1, wherein, in the formula I, X are identical or different and are phenyl, tolyl or naphthyl radicals and $R^1$ is hydrogen, a methyl, isopropyl, isobutyl, t-butyl, phenyl or naphthyl radical, m is 1 and n is 0 or 1.

**3.** The process as claimed in claim 1 or 2, wherein, in the formula II, A is a straight-chain or branched $C_8$-$C_{20}$-, in particular $C_{10}$-$C_{16}$-alkyl, $C_8$-$C_{20}$-, in particular $C_{10}$-$C_{18}$-ω-hydroxyalkyl or a substituted or unsubstituted $C_6$-$C_{18}$-, in particular $C_6$-$C_{12}$-aryl radical or the radical $R^7$-CONH-$CH_2$-$CH_2$-$CH_2$-, where $R^7$ is a straight-chain or branched alkyl radical having from 4 to 10, in particular from 3 to 9, carbon atoms, B, C and D are identical or different and are straight-chain or branched alkyl or ω-hydroxyalkyl radicals having 2 or 3 carbon atoms or C and D together with N form a heterocyclic six-membered ring.

**4.** The process as claimed in one or more of claims 1 to 3, wherein $E^-$ in formula II is a halide, sulfate, methosulfate, sulfonate or borate ion.

**5.** The process as claimed in claim 4, wherein $E^-$ in formula II is chloride, bromide, iodide, benzenesulfonate, $C_7$-$C_{10}$-alkylbenzenesulfonate, in particular toluenesulfonate or tetrafluoroborate.

**6.** The process as claimed in one or more of claims 1 to 3, wherein $E^-$ in formula II is a carboxylate ion, preferably acetate ion, a lactate ion or a citrate ion.

**7.** The process as claimed in one or more of claims 1 and 3-6, wherein the ligands used in the water-soluble rhodium complexes are sulfonated 2,2'-bis(diphenylphosphinomethyl)-1,1'-binaphthyls of the formula III

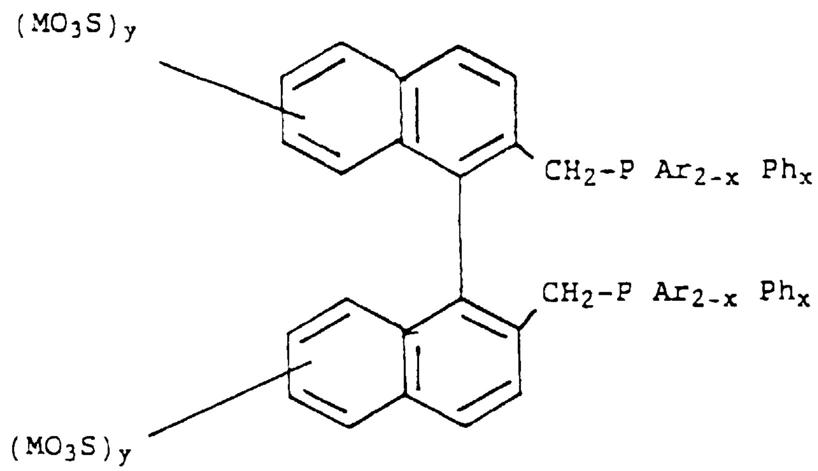

III

where Ar is m-$C_6H_4SO_3M$, M is hydrogen, ammonium, a monovalent metal or the equivalent of a polyvalent metal, preferably lithium, sodium, potassium or barium, and Ph is the phenyl radical, y are identical or different and are 1 or 2, preferably 2, and x are identical or different and are 0, 1 or 2, preferably 1 or 2.

**8.** The process as claimed in one or more of claims 1 to 7, wherein the concentration of the solubilizer in the aqueous catalyst solution is from 0.05 to 5% by weight, preferably 0.07-2% by weight and particularly preferably 0.1-0.5% by weight, based on the catalyst solution.

**9.** The process as claimed in one or more of claims 1 to 8, wherein the rhodium concentration in the aqueous catalyst solution is from 10 to 2000 ppm by weight, preferably from 20 to 300 ppm by weight and particularly preferably from 40 to 100 ppm by weight, based on the catalyst solution, and 1-50 mol, preferably 5-15 mol, of diphosphine are used per mol of rhodium.

**10.** The process as claimed in one or more of claims 1 to 9, wherein the reaction is carried out at temperatures of from 20 to 150°C, preferably 50-120°C, and pressures of from 0.1 to 20 MPa, preferably 1-10 MPa.

**11.** The process as claimed in one or more of claims 1-10, wherein the olefinically unsaturated compounds used are substituted or unsubstituted alkenes having from 3 to 20 carbon atoms, substituted or unsubstituted dienes having from 4 to 10 carbon atoms, substituted or unsubstituted cycloalkenes or dicycloalkenes having from 5 to 12 carbon atoms in the ring system, esters of an unsaturated carboxylic acid having from 3 to 20 carbon atoms and an aliphatic alcohol having from 1 to 18 carbon atoms, esters of a saturated carboxylic acid having from 2 to 20 carbon atoms and an unsaturated alcohol having from 2 to 18 carbon atoms, unsaturated alcohols or ethers each having from 3 to 20 carbon atoms or araliphatic olefins having from 8 to 20 carbon atoms.

## Revendications

**1.** Procédé de préparation d'aldéhydes par réaction de composés à insaturation oléfinique d'au moins 3 atomes de carbone avec du monoxyde de carbone et de l'hydrogène en phase liquide en présence d'eau, d'un système de catalyseur contenant des complexes de rhodium solubles dans l'eau et d'agents de solubilisation, caractérisé en ce que les complexes de rhodium solubles dans l'eau contenus dans le système de catalyseur contiennent comme ligands des diphosphines de formule générale I

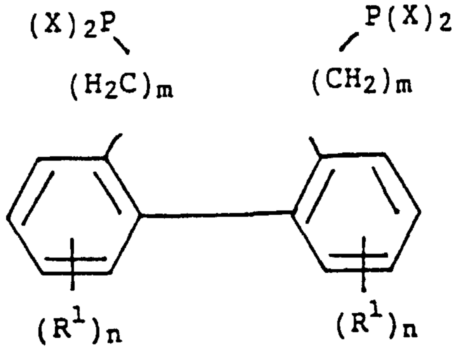

I

substituées par un ou plusieurs groupes acide sulfonique, dans lesquelles les X sont identiques ou différents et représentent des restes alkyle en $C_1$-$C_9$, cycloalkyle en $C_6$-$C_{10}$, aryle en $C_6$-$C_{10}$ substitués ou non substitués ou biaryle en $C_{12}$, les $R^1$ sont identiques ou différents et représentent l'hydrogène ou des restes alkyle en $C_1$-$C_{14}$, alcoxy en $C_1$-$C_{14}$, cycloalkyle en $C_6$-$C_{14}$, aryle en $C_6$-$C_{14}$ ou aryloxy en $C_6$-$C_{14}$, ou un cycle benzène condensé, les m sont identiques ou différents et représentent des nombres entiers de 0 à 5 et les n sont également identiques ou différents et représentent des nombres entiers de 0 à 4,
et en ce que l'on utilise comme agents de solubilisation des composés de formule générale II:

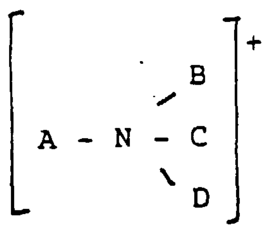

$E^-$ II

dans laquelle A est un reste alkyle en $C_6$-$C_{25}$ ou ω-hydroxyalkyle en $C_6$-$C_{25}$, linéaire ou ramifié, un reste aryle en $C_6$-$C_{25}$ éventuellement substitué, ou le reste $R^7$-CONH-$CH_2$-$CH_2$-$CH_2$, où $R^7$ est un reste alkyle linéaire ou ramifié de 5 à 11 atomes de carbone, B, C et D sont identiques ou différents et représentent des restes alkyle ou ω-hydroxyalkyle linéaires ou ramifiés de 1 à 4 atomes de carbone, ou C et D forment ensemble avec N un hétérocycle de 5 ou 6 chaînons, et $E^-$ représente un anion inorganique ou organique.

2. Procédé selon la revendication 1, caractérisé en ce que, dans la formule générale I, les X sont identiques ou différents et représentent des restes phényle, tolyle ou naphtyle, et $R^1$ représente l'hydrogène ou un reste méthyle, isopropyle, isobutyle, t-butyle, phényle ou naphtyle, m est égal à 1 et n est égal à 0 ou 1.

3. Procédé selon la revendication 1 ou 2, caractérisé en ce que, dans la formule générale II, A représente un reste alkyle en $C_8$-$C_{20}$, en particulier en $C_{10}$-$C_{16}$, ou ω-hydroxyalkyle en $C_8$-$C_{20}$, en particulier en $C_{10}$-$C_{18}$, linéaire ou ramifié, ou un reste aryle éventuellement substitué en $C_6$-$C_{18}$, en particulier en $C_6$-$C_{12}$, ou le reste $R^7$-CONH-$CH_2$-$CH_2$-$CH_2$ dans lequel $R^7$ est un reste alkyle linéaire ou ramifié de 4 à 10, en particulier de 3 à 9 atomes de carbone, B, C et D sont identiques ou différents et représentent des restes alkyle ou ω-hydroxyalkyle linéaires ou ramifiés de 2 à 3 atomes de carbone, ou C et D forment ensemble avec N un hétérocycle de 6 chaînons.

4. Procédé selon l'une ou plusieurs des revendications 1 à 3, caractérisé en ce que, dans la formule II, $E^-$ représente un ion halogénure, sulfate, méthylsulfate, sulfonate ou borate.

5. Procédé selon la revendication 4, caractérisé en ce que, dans la formule II, $E^-$ représente un ion chlorure, bromure, iodure, benzènesulfonate, alkylbenzènesulfonate en $C_7$-$C_{10}$, en particulier toluènesulfonate, ou tétrafluoroborate.

6. Procédé selon l'une ou plusieurs des revendications 1 à 3, caractérisé en ce que, dans la formule II, $E^-$ représente un ion carboxylate, de préférence acétate, un ion lactate ou un ion citrate.

**7.** Procédé selon l'une ou plusieurs des revendications 1 et 3 à 6, caractérisé en ce que l'on utilise comme ligands des complexes de rhodium solubles dans l'eau des 2,2'-bis(diphénylphosphinométhyl)-1,1'-binaphtyles sulfonés de formule III:

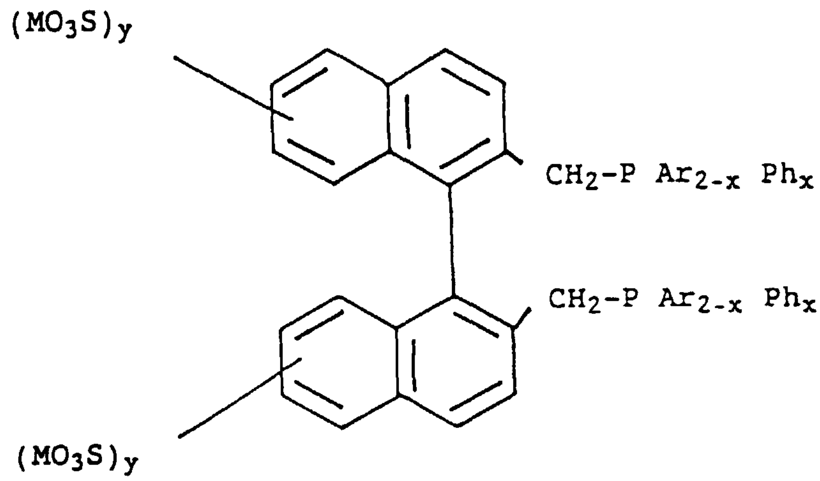

III

dans laquelle Ar est un reste m-$C_6H_4SO_3M$, M représente l'hydrogène, l'ammonium, un métal monovalent ou l'équivalent d'un métal polyvalent, de préférence le lithium, le sodium, le potassium ou le baryum, et Ph représente le reste phényle, les y sont identiques ou différents et valent 1 ou 2, de préférence 2, et les x sont identiques ou différents et valent 0, 1 ou 2, de préférence 1 ou 2.

**8.** Procédé selon l'une ou plusieurs des revendications 1 à 7, caractérisé en ce que la concentration de l'agent de solubilisation dans la solution aqueuse de catalyseur est de 0,05 à 5 % en masse, de préférence de 0,07 à 2 % en masse et de façon particulièrement préférée de 0,1 à 0,5 % en masse, par rapport à la solution de catalyseur.

**9.** Procédé selon l'une ou plusieurs des revendications 1 à 8, caractérisé en ce que la concentration de rhodium dans la solution aqueuse de catalyseur est de 10 à 2 000 ppm en masse, de préférence de 20 à 300 ppm en masse et de façon particulièrement préférée de 40 à 100 ppm en masse, par rapport à la solution de catalyseur, et en ce que l'on utilise 1 à 50 mol, de préférence 5 à 15 mol de diphosphine par mole de rhodium.

**10.** Procédé selon l'une ou plusieurs des revendications 1 à 9, caractérisé en ce que la réaction s'effectue à des températures de 20 à 150°C, de préférence de 50 à 120°C, et sous des pressions de 0,1 à 20 MPa, de préférence de 1 à 10 MPa.

**11.** Procédé selon l'une ou plusieurs des revendications 1 à 10, caractérisé en ce que l'on utilise comme composés à insaturation oléfinique des alcènes substitués ou non substitués de 3 à 20 atomes de carbone, des diènes substitués ou non substitués de 4 à 10 atomes de carbone, des cycloalcènes ou des dicycloalcènes substitués ou non substitués de 5 à 12 atomes de carbone dans le système cyclique, des esters d'un acide carboxylique insaturé de 3 à 20 atomes de carbone et d'un alcool aliphatique de 1 à 18 atomes de carbone, des esters d'un acide carboxylique saturé de 2 à 20 atomes de carbone et d'un alcool insaturé de 2 à 18 atomes de carbone, des alcools ou des éthers insaturés ayant chacun 3 à 20 atomes de carbone ou des oléfines araliphatiques de 8 à 20 atomes de carbone.